# EUROPÄISCHE PATENTANMELDUNG

(11) **EP 1 475 636 A1**
(43) Veröffentlichungstag der Anmeldung: **10.11.2004**
(21) Anmeldenummer: 04009916.0
(22) Anmeldetag: 27.04.2004
(51) Int. Cl.: G01N 33/00, G01N 27/407, G01N 27/406, G01N 27/417, G01N 27/419

(54) **Verfahren zur Bestimmung der Konzentration einer Gaskomponente im Abgas einer Brennkraftmaschine**

(30) Priorität: 07.05.2003 DE 10320257
(71) Anmelder: DaimlerChrysler AG, 70567 Stuttgart (DE)
(72) Erfinder: Hofmann, Uwe, 64385 Reichelsheim (DE)

(57) **Zusammenfassung**

Es wird ein Verfahren zur Bestimmung eines Gehalts einer vorgegebenen ersten Gaskomponente in einem Abgas einer Brennkraftmaschine (1) stromab eines in einer Abgasleitung (3) der Brennkraftmaschine (1) angeordneten Abgaskatalysators (4) vorgeschlagen, wobei ein an ein elektronisches Steuergerat (7) angeschlossener erster Gassensor (5) von wenigstens einem Teil des aus dem Abgaskatalysator (4) austretenden Abgases angeströmt wird.

Erfindungsgemäß ist ein an das elektronische Steuergerät (7) angeschlossener zweiter Gassensor (6) vorgesehen, der von wenigstens einem Teil des aus demselben Abgaskatalysator (4) austretenden Abgases angeströmt wird und der Gehalt der ersten Gaskomponente vom Steuergerät (7) wird durch eine Verknüpfung eines Signals des ersten Gassensors (5) mit einem Signal des zweiten Gassensors (6) ermittelt.

Das Verfahren ist insbesondere zur Ermittlung des Stickoxidgehalts im Abgas der Brennkraftmaschine (1) eines Kraftfahrzeugs anwendbar.

## Beschreibung

Die Erfindung betrifft ein Verfahren zur Bestimmung des Gehalts einer vorgegebenen Gaskomponente in einem Abgas einer Brennkraftmaschine mit den Merkmalen des Oberbegriffs des Anspruchs 1.

In der Europäischen Patentanmeldung EP 1 060 003 B1 ist ein Verfahren zur Bestimmung eines Gehalts von Stickoxiden in einem Abgas einer Brennkraftmaschine stromab eines in einer Abgasleitung der Brennkraftmaschine angeordneten Abgaskatalysators mittels eines Stickoxid-Sensors beschrieben. Das Signal des Stickoxid-Sensors wird dabei für eine Umsteuerung des Luft-Kraftstoffverhältnisses der Brennkraftmaschine zum Zwecke einer Regenerierung des Abgaskatalysators von einem elektronischen Steuergerät ausgewertet. Der eingesetzte Stickoxid-Sensor weist gegenüber einer reduzierenden Abgaskomponente eine Querempfindlichkeit auf, die bei dem Verfahren gezielt ausgenutzt wird. Dennoch besteht das grundsätzliche Problem der zuverlässigen Interpretation des Signals des eingesetzten Stickoxid-Sensors.

Aufgabe der Erfindung ist es, ein Verfahren anzugeben, welches eine zuverlässige Ermittlung eines Gehalts einer vorgegebenen Gaskomponente in einem Abgas einer Brennkraftmaschine ermöglicht.

Die Aufgabe wird erfindungsgemäß durch ein Verfahren mit den Merkmalen des Anspruchs 1 gelöst.

Das erfindungsgemäße Verfahren ist dadurch gekennzeichnet, dass analog zu einem ersten Gassensor ein zweiter Gassensor an das elektronische Steuergerät angeschlossen ist und wenigstens von einem Teil des aus dem Abgaskatalysator austretenden Abgases angeströmt wird und der Gehalt der ersten Gaskomponente vom Steuergerät durch eine Verknüpfung eines Signals des ersten Gassensors mit einem Signal des zweiten Gassensors ermittelt wird. Vorzugsweise wird die Verknüpfung nur zeitweise vorgenommen und erfolgt in Abhängigkeit vom Signal des ersten Gassensors und/oder in Abhängigkeit vom Signal des zweiten Gassensors. Durch das erfindungsgemäße Verfahren kann die Genauigkeit und Zuverlässigkeit bei der Ermittlung des Gehalts der ersten Gaskomponente beispielsweise dadurch verbessert werden, dass das Signal des ersten Gassensors in einem Wertebereich korrigiert wird, in welchem er ungenau oder unzuverlässig arbeitet. Zu diesem Zweck wird das Signal des ersten Gassensors mit dem des zweiten Gassensors verknüpft. Die Verknüpfung der Signale der Gassensoren kann dabei sowohl durch logische als auch durch arithmetische Operationen erfolgen. Die Gassensoren können hierbei überwiegend auf dieselbe erste Gaskomponente oder auf verschiedene Gaskomponenten empfindlich sein. Vorzugsweise werden der erste und der zweite Gassensor von Abgas gleicher Zusammensetzung angeströmt, wozu die Sensoren, vorzugsweise im Abgasstrang, entsprechend anzuordnen sind, beispielsweise ausgangsseitig des Abgaskatalysators. Mit dem erfindungsgemäßen Verfahren können somit aus unterschiedlichen Empfindlichkeiten oder unterschiedlichen Querempfindlichkeiten der Gassensoren resultierende Ungenauigkeiten überwunden werden.

In Ausgestaltung der Erfindung weisen der erste Gassensor und der zweite Gassensor unterschiedliche Kennlinien auf. Insbesondere im Falle von Gassensoren, die überwiegend gegenüber derselben ersten Gaskomponente empfindlich sind, kann damit beispielsweise eine in einem Messbereich vorhandene mangelnde Empfindlichkeit des einen Sensors mit Hilfe des Signals des anderen Sensors kompensiert werden, so dass insgesamt eine zuverlässigere Messung resultiert. Auf diese Weise kann insbesondere mit Vorteil in Konzentrationsbereichen der zu bestimmenden ersten Gaskomponente verfahren werden, in welchen die Gassensoren unterschiedlich steile Kennlinienverläufe aufweisen.

In weiterer Ausgestaltung der Erfindung korreliert das Signal des ersten Gassensors mit dem Partialdruck der ersten Gaskomponente und das Signal des zweiten Gassensors korreliert mit dem Partialdruck einer zweiten, zur ersten Gaskomponente unterschiedlichen Gaskomponente. Die Gassensoren weisen demnach überwiegend eine Empfindlichkeit gegenüber verschiedenen Gaskomponenten auf. Vorzugsweise wird durch den zweiten Sensor die Gegenwart einer zweiten Gaskomponente erkannt, welche die Anzeigegenauigkeit des ersten Gassensors beeinflusst. Dieser Einfluss kann dann durch das Steuergerät korrigiert werden, was die Zuverlässigkeit bei der Interpretation des Signals des ersten Sensors verbessert.

In weiterer Ausgestaltung der Erfindung korreliert das Signal des ersten Gassensors wenigstens mit dem Partialdruck von Stickoxid, und das Signal des zweiten Gassensor korreliert mit dem Partialdruck von Sauerstoff, und vom Steuergerät wird durch Verknüpfung des Signals des ersten Gassensors mit dem Signal des zweiten Gassensors der Stickoxidgehalt im Abgas ermittelt. Beim ersten Gassensor handelt es sich vorzugsweise um einen Stickoxid-Sensor mit einer Querempfindlichkeit gegenüber anderen Gaskomponenten, insbesondere in reduzierender Atmosphäre. Das Vorliegen einer reduzierenden Atmosphäre kann jedoch über das Signal des gegenüber Sauerstoff empfindlichen zweiten Gassensors erkannt werden. In diesem Fall wird vom Steuergerät das Signal des ersten, gegenüber Stickoxiden empfindlichen Gassensors über eine logische und/oder arithmetische Verknüpfung mit dem Signal des zweiten, gegenüber Sauerstoff empfindlichen Gassensors korrigiert und somit ein zuverlässigerer Wert für den Stickoxidgehalt im Abgas gewonnen.

In weiterer Ausgestaltung der Erfindung wird die Verknüpfung der Signale der Gassensoren derart vorgenommen, dass unterhalb eines vorgebbaren Schwellenwertes S1 für das Signal des zweiten Gassensors das Signal des ersten Gassensors mit einem vorgebbaren ersten Faktor F1 multipliziert wird ,und oberhalb des Schwellenwertes S1 für das Signal des zweiten Gassensors das Signal des ersten Gassensors mit einem vorgebbaren zweiten Faktor F2 multipliziert wird. Vorzugsweise wird der Schwellenwert S1 so festgelegt, dass bei der entsprechenden Abgaszusammensetzung der erste Gassensor eine starke Änderung seiner Empfindlichkeit gegenüber einer oder mehreren Abgaskomponenten aufweist. Vorteilhaft ist diese Vorgehensweise insbesondere, wenn ein erster und ein zweiter Gassensor eingesetzt werden, die gegenüber unterschiedlichen Gaskomponenten empfindlich sind. Für die Faktoren F1, F2 können feste Werte vorgesehen sein. Die Faktoren F1, F2 können jedoch auch eine Abhängigkeit vom Signalwert des zweiten Gassensors aufweisen, welche unterhalb des Schwellenwertes S1 anders geartet ist als oberhalb. Weiter kann der Schwellenwert S1 auch abhängig davon gewählt werden, ob das Signal des ersten Gassensors und/oder das Signal des zweiten Gassensors aktuell ansteigt oder absinkt. Insbesondere ist gemäß einer weiteren Ausgestaltung der Erfindung vorgesehen, dass die Verknüpfung nur dann vorgenommen wird, wenn das Signal des ersten Gassensors ein zumindest lokales Maximum überschritten hat. Die Durchführung der Verknüpfung kann dann bei Vorliegen einer bestimmten Bedingung, wie beispielsweise nach Unterschreiten eines vorgebbaren Schwellenwertes für das Signal des ersten Gassensors, wieder beendet werden.

In weiterer Ausgestaltung der Erfindung wird die Verknüpfung der Gassensorsignale vorgenommen, wenn das Signal des zweiten Gassensors einen Wert innerhalb eines vorgebbaren Wertebereichs aufweist. Da vorzugsweise der Wertebereich des Signals des zweiten Gassensors dem Konzentrationsbereich einer Gaskomponente entspricht, bei welcher Anzeigeungenauigkeiten des ersten Gassensors vorhanden sind, kann das Signal des ersten Gassensors mit Hilfe des Signals des zweiten Gassensors korrigiert werden. Dadurch wird die Zuverlässigkeit bei der Interpretation des Signals des ersten Gassensors verbessert.

In weiterer Ausgestaltung der Erfindung wird durch Integration des ermittelten Gehalts der ersten Gaskomponente über einen vorgebbaren Zeitbereich hinweg die Gesamtemission der ersten Gaskomponente in dem Zeitbereich ermittelt. Durch die erfindungsgemäße Verknüpfung der Signale der Gassensoren wird ein zuverlässigerer Wert für den Gehalt der ersten Gaskomponente im Abgas gewonnen. Dementsprechend zuverlässig wird auch die durch Integration gewonnene Gesamtemission, so dass insgesamt eine verbesserte Aussage über die Emission eines der Brennkraftmaschine zugeordneten Kraftfahrzeugs ermöglicht ist.

In weiterer Ausgestaltung der Erfindung wird bei Überschreiten eines vorgebbaren Schwellenwertes E1 für die Gesamtemission der ersten Gaskomponente vom Steuergerät eine Meldung erzeugt. Dies ist insbesondere von Bedeutung, wenn es sich bei der ersten Gaskomponente um einen Schadstoff wie beispielsweise Stickoxid handelt. In diesem Fall kann eine unzulässig hohe Schadstoffemission zuverlässig erkannt und angezeigt werden. Auf diese Weise kann beispielsweise die Funktion des Abgaskatalysators überwacht werden und im Rahmen einer onboard-Diagnose eine Fehlfunktion des Katalysators erkannt und gemeldet werden.

Im Folgenden wird die Erfindung anhand von Zeichnungen und zugehörigen Beispielen näher erläutert. Dabei zeigen:
- Fig. 1: schematisch dargestellt eine Anordnung zur Durchführung des erfindungsgemäßen Verfahrens und
- Fig. 2: ein Diagramm mit schematisch dargestellten Signalverläufen zur Erläuterung des Verfahrensprinzips.

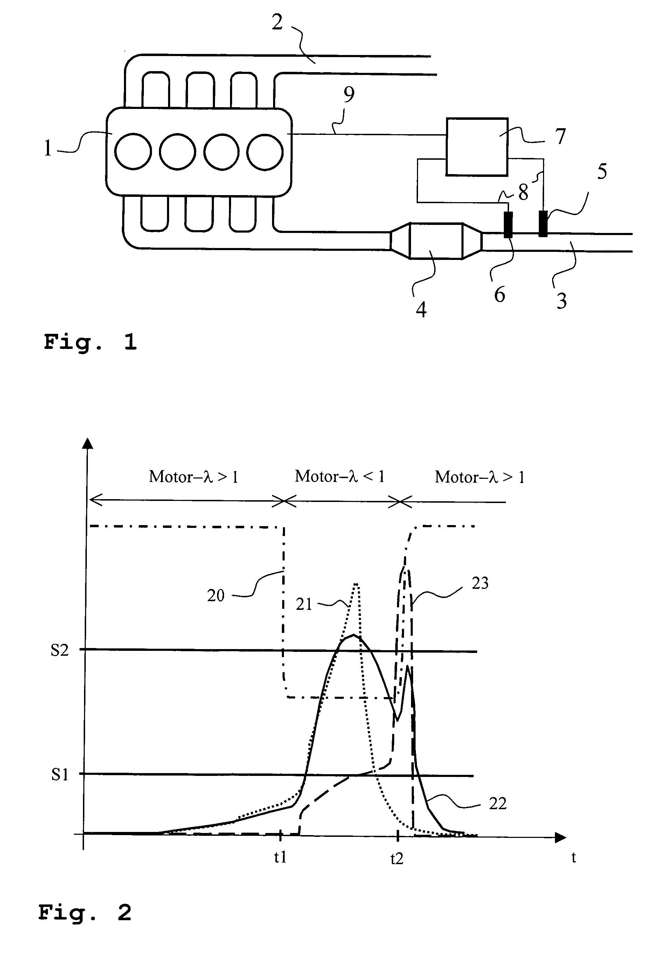

In Fig. 1 ist eine Brennkraftmaschine 1 mit einer Ansaugluftleitung 2 und einer Abgasleitung 3 dargestellt. In der Abgasleitung 3 ist ein Abgaskatalysator 4 angeordnet. Ausgangsseitig des Abgaskatalysators 4 sind ein erster Gassensor 5 und ein zweiter Gassensor 6 derart in der Abgasleitung 3 angeordnet, dass sie von Abgas mit gleicher oder annähernd gleicher Zusammensetzung angeströmt sind. Die Abgassensoren 5, 6 sind über Signalleitungen 8 an ein elektronisches Steuergerät 7 angeschlossen, das zur Steuerung des Brennkraftmaschinenbetriebs außerdem über eine Steuerleitung 9 an die Brennkraftmaschine 1 angeschlossen ist. Ohne Einschränkung der Allgemeingültigkeit wird im Folgenden davon ausgegangen, dass die Brennkraftmaschine 1, nachfolgend kurz als Motor bezeichnet, in einem Mager-Fett-Wechselbetrieb betrieben wird und der Abgaskatalysator 4 als sogenannter Stickoxidspeicher-Katalysator, kurz Katalysator genannt, ausgebildet ist. Im nachfolgend näher erläuterten Beispiel wird ferner davon ausgegangen, dass der erste Abgassensor 5 als Stickoxidsensor, kurz NOx-Sensor genannt und der Abgassensor 6 als Sauerstoffsensor, kurz λ-Sonde genannt, ausgebildet ist.

In der verbrauchsgünstigen mageren Phase des Mager-Fett-Wechselbetriebs des Motors 1 entzieht das als Speicherkomponente im Katalysator 4 beispielsweise vorhandene Bariumcarbonat dem dann oxidierenden Abgas Stickoxid (NOx) unter Bildung von Bariumnitrat. Auf Grund der damit verbundenen Materialerschöpfung wird von Zeit zu Zeit eine Regeneration des Katalysators 4 notwendig. Diese sogenannte Nitratregeneration geschieht dadurch, dass der Motor 1 für eine gewisse Zeit fett betrieben wird. Das in dem resultierenden reduktionsmittelhaltigen und reduzierend wirkenden Abgas instabile Bariumnitrat zersetzt sich hierbei wieder unter Rückbildung von Bariumcarbonat und unter Freisetzung von NOx. Letzteres wird von den dann im Abgas vorhandenen Reduktionsmitteln an der auf dem Katalysator 4 aufgebrachten Edelmetallkomponente überwiegend zu unschädlichem Stickstoff reduziert. Die Nitratregeneration wird eingeleitet, wenn die Aufnahmefähigkeit des Speichermaterials in der Magerphase soweit abgesunken ist, dass ein unerwünscht hoher NOx-Schlupf auftritt, welcher vom NOx-Sensor 5 messtechnisch erfasst und als solcher vom Steuergerät 7 erkannt wird. Unter Berücksichtigung des Abgasmassenstroms und weiterer dem Steuergerät 7 zur Verfügung stehender Größen sowie des Ausgangssignals des NOx-Sensors 5 kann vom Steuergerät 7 die in der Magerphase des Mager-Fett-Wechselbetriebs des Motors 1 aus dem Katalysator 4 strömende NOx-Menge ermittelt werden.

Neben dem NOx-Schlupf des Katalysators 4 in der Magerphase tritt jedoch auch häufig ein mehr oder weniger starker NOx-Schlupf im Fettbetrieb der Nitratregeneration, meist in Form einer kurzen NOx-Desorptionsspitze auf. Vermutliche Ursache dieser Desorptionsspitze ist, dass die Zersetzung der Nitrate unter reduzierenden Bedingungen schneller abläuft als deren katalytisch unterstützte Reduktion zu Stickstoff bzw. Ammoniak an der auf dem Katalysator 4 vorhandenen Edelmetallkomponente. Da der NOx-Sensor 5 jedoch, insbesondere unter reduzierenden Bedingungen, eine gewisse Querempfindlichkeit gegenüber reduzierenden Abgaskomponenten wie Ammoniak (NH3) aufweist, kann in diesem Fall allein anhand des Signals des NOx-Sensors 5 nicht entschieden werden, ob bzw. inwieweit das vom NOx-Sensor 5 unter reduzierenden Bedingungen gelieferte Signal von einem NOx-Anteil im Abgas oder beispielsweise von einem NH3-Anteil im Abgas verursacht wird. Die zur Einhaltung niedriger Emissionswerte erforderliche Bilanzierung der insgesamt im Mager-Fett-Wechselbetrieb des Motors 1 aus dem Katalysator ist aus diesem Grunde ohne zusätzliche Maßnahmen nicht möglich.

Erfindungsgemäß wird jedoch während des Mager-Fett-Wechselbetriebs zusätzlich das Signal der λ-Sonde 6 ausgewertet. Durch Verknüpfung der Signale des NOx-Sensors 5 und der λ-Sonde 6 kann eine zuverlässige Bewertung des Signals des NOx-Sensors 5 vom Steuergerät 7 vorgenommen werden und somit die NOx-Emission zuverlässig im gesamten Mager-Fett-Betrieb vorgenommen werden.

Eine hierzu vorteilhafte Vorgehensweise wird nachfolgend beispielhaft anhand des in der Fig. 2 dargestellten Diagramms und unter Bezugnahme auf die Fig. 1 erläutert.

Im Diagramm der Fig. 2 sind ausschnittsweise zeitliche Signalverläufe während eines Mager-Fett-Wechselbetriebs des Motors dargestellt. Dabei gibt die Spur 20 den Verlauf des Luft-Kraftstoffverhältnisses an, mit welchem der Motor 1 betrieben wird. Dieses Luft-Kraftstoffverhältnis wird nachfolgend als Motor-λ bezeichnet. Zum Zeitpunkt t1 wechselt das Motor-λ von einem Wert größer als eins zu einem Wert kleiner als eins, wodurch die Nitratregeneration des Katalysators 4 eingeleitet wird. Zum Zeitpunkt t2 wird die Nitratregeneration beendet und das Motor-λ wird vom Steuergerät 7 wieder auf einen Wert größer als eins zurückgestellt. Dabei resultiert aus dem unkorrigierten Signal des NOx-Sensors 5 der durch die Spur 22 dargestellte gemessene Verlauf der NOx-Konzentration. Demgegenüber wird der korrekte Verlauf der tatsächlich vorhandenen NOx-Konzentration ausgangsseitig des Katalysators 4 durch die Spur 21 wiedergegeben. Es ist deutlich zu erkennen, dass zwar etwa bis zur Mitte des durch t1 und t2 gegebenen Nitratregenerationsintervalls die NOx-Konzentration durch den NOx-Sensor 5 richtig wiedergegeben wird. Etwa ab der Mitte des Nitratregenerationsintervalls liefert das unkorrigierte Signal des NOx-Sensors 5 im Vergleich zu den tatsächlichen Verhältnissen jedoch zu große NOx-Konzentrationen, was demgemäss eine Fehlmessung darstellt.

Ursache hierfür ist eine Querempfindlichkeit des NOx-Sensors 5 gegenüber reduzierenden Abgasbestandteilen wie Ammoniak (NH3), Wasserstoff (H2), Kohlenmonoxid (CO) oder Kohlenwasserstoffen (HC). Insbesondere CO, H2 und HC sind mit zunehmender Regenerationsdauer in zunehmendem Maße ausgangsseitig des NOx-Katalysators 4 im Abgas vorhanden, da ihnen mit fortschreitender Regeneration des Katalysators 4 immer weniger Reaktionspartner im Katalysator 4 zur Verfügung stehen. Daneben kann es auch zu einer Reduktion von aus dem Speichermaterial des Katalysators 4 freigesetztem NOx zu NH3 kommen. Infolgedessen macht sich das eingangsseitig des Katalysators 4 eingestellte Motor-λ auch ausgangsseitig'des Katalysators 4 durch Absinken des sogenannten Abgas-λ bemerkbar, was mit der λ-Sonde 6 messtechnisch erfasst werden kann. Der Signalverlauf der hier als sogenannte Binärsonde ausgeführten λ-Sonde 6 wird im Diagramm der Fig. 2 durch die Spur 23 wiedergegeben, wobei große Signalwerte einem kleinen Abgas-λ-Wert zugeordnet sind und umgekehrt. Der gegen Ende des Regenerationsintervalls stark ansteigende Signalwert (Spur 23) der λ-Sonde 6 repräsentiert folglich ein absinkendes, sich an das eingestellte Motor-λ annähernde Abgas-λ. Damit kann die Nitratregeneration des Katalysators 4 als beendet betrachtet werden, und vom Steuergerät 7 wird zum Zeitpunkt t2, getriggert durch das ansteigende Signal der λ-Sonde 6, wieder ein mageres Motor-λ, d.h. ein Motor-λ größer als 1 eingestellt. Dies macht sich ausgangsseitig des Katalysators 4 vergleichsweise rasch bemerkbar, weshalb das durch die Spur 23 gegebene Signal der λ-Sonde 6 entsprechend einem Abgas-λ größer als 1 wieder absinkt.

Erfindungsgemäß wird das Signal der λ-Sonde 6 zusätzlich zur Triggerung der Motor-λ-Umstellung zur Bestimmung des NOx-Gehalts im Abgas herangezogen. Zu diesem Zweck wird es vom Steuergerät 7 mit dem Signal des NOx-Sensors 5 verknüpft.

Als Verknüpfung kann beispielsweise vorgesehen sein, dass unterhalb eines vorgebbaren Schwellenwertes S1 für das Signal der λ-Sonde 6 das Signal des NOx-Sensors 5 mit einem vorgebbaren ersten Faktor F1 multipliziert wird und oberhalb des Schwellenwertes S1 für das Signal der λ-Sonde 6 das Signal des NOx-Sensors 5 mit einem vorgebbaren zweiten Faktor F2 multipliziert wird. Eine ausreichende Genauigkeit für die ermittelte NOx-Konzentration im Abgas wird bereits erhalten, wenn der Faktor F1 gleich eins gewählt wird und der Faktor F2 gleich Null gewählt wird. Wird der Faktor F1 gleich eins gewählt, entspricht dies einer nur zeitweise vorgenommenen Verknüpfung der Signale. Die Genauigkeit kann weiter verbessert werden, wenn der Faktor F1 und/oder der Faktor F2 in Abhängigkeit vom Signalwert der λ-Sonde 6 gewählt werden. Beispielsweise ist es günstig, für den Faktor F2 eine umgekehrt proportionale Abhängigkeit zum Signalwert der λ-Sonde 6 zu wählen.

Es kann ferner vorgesehen sein, die Durchführung der Signalwert-Verknüpfung von weiteren und/oder anderen Bedingungen abhängig zu machen. So kann es vorteilhaft sein, das Signal des NOx-Sensors 5 mit dem Faktor F2 zu multiplizieren, wenn der Schwellenwert S1 für das Signal der λ-Sonde 6 überschritten ist und gleichzeitig das Signal des NOx-Sensors 5 ein zumindest lokales Maximum überschritten hat. Im dargestellten Beispiel wird zweckmäßigerweise gefordert, dass dieses Maximum innerhalb des zwischen t1 und t2 liegenden Regenerationsintervalls liegen muss.

Als alternative Verknüpfungsmöglichkeit kann vorgesehen sein, das Signal des NOx-Sensors 5 mit dem Faktor F2 zu multiplizieren, wenn das Signal der λ-Sonde 6 innerhalb eines durch vorgebbare Schwellenwerte S1 und S2 definierten Wertebereiches liegt, und gleichzeitig das Signal des NOx-Sensors 5 ein zumindest lokales Maximum uberschritten hat.

Die Größe der Schwellenwerte S1 und S2 können selbstverständlich von weiteren Größen wie beispielsweise von der Abgastemperatur und/oder dem Abgasmassenstrom abhängig sein und für ein ansteigendes Signal der λ-Sonde 6 anders gewählt werden als für ein absinkendes Signal.

Es ist klar, dass auch andere hier nicht explizit genannte Verknüpfungen der Signale der λ-Sonde 6 und des NOx-Sensors 5 vorteilhaft sein können und zu einer verbesserten Genauigkeit für den ermittelten NOx-Gehalt im Abgas führen.

Die durch die Signalverknüpfung erzielte verbesserte Genauigkeit für den ermittelten NOx-Gehalt im Abgas erlaubt es, die Gesamtemission von NOx durch zeitliche Integration der Werte für den NOx-Gehalt im Abgas mit großer Genauigkeit durchzuführen. Die Integration liefert dann, gegebenenfalls nach entsprechenden Umrechnungen, Aussagen hinsichtlich der NOx-Emission in g je km Fahrstrecke oder in g je kwh Motorarbeit. Die hierfür zusätzlich notwendige Kenntnis des gesamten Abgasmassenstroms ist durch die dem Steuergerät 7 zur Verfügung stehenden Werte für den Luftdurchsatz und den Kraftstoffdurchsatz gegeben. Dasselbe gilt für die im Integrationsintervall zurückgelegte Fahrstrecke. Die Ermittlung der Gesamtemission von NOx kann dabei laufend oder in ausgewählten Betriebsbereichen des Motors 1 vorgenommen werden.

Mit der ermittelten Gesamtemission von NOx kann schließlich mit Vorteil auf die Funktionsfähigkeit des Katalysators 4 geschlossen werden. Wird beispielsweise festgestellt, dass die NOx-Gesamtemission einen vorgebbaren Emissionsschwellenwert E1 überschreitet, so kann Einfluss auf den Motorbetrieb genommen werden, um die NOx-Emission zu verbessern. So kann beispielsweise vorgesehen sein, bei Überschreiten des Emissionsschwellenwertes E1 auf einen Motorbetrieb mit λ gleich 1 überzugehen, der üblicherweise für die NOx-Emission unkritisch ist. Es kann jedoch auch vorgesehen sein, eine Schwefelregeneration des Katalysators 4 einzuleiten. Ferner kann vorgesehen sein, die Magerphasen des Mager-Fett-Wechselbetriebs zu verkürzen, um den Katalysator 4 weniger zu belasten. Schließlich kann bei entsprechend großer Überschreitung des Emissionsschwellenwertes E1 eine Warnung ausgegeben werden, welche auf die erhöhte NOx-Emission bzw. auf einen geschädigten Katalysator 4 hinweist. Die genannten Maßnahmen können selbstverständlich in Abhängigkeit von der Stärke der Überschreitung des Emissionsschwellenwertes E1 ausgewählt werden.

Es versteht sich, dass das erfindungsgemäße Verfahren auch auf andere Kombinationen von Gassensoren anwendbar ist. Beispiele hierfür sind zwei verschiedene λ-Sonden, wie eine λ-Breitbandsonde und eine λ-Sprungsonde mit abschnittsweise stark unterschiedlichen Kennlinienverläufen bzw. Empfindlichkeiten. Das erfindungsgemäße Verfahren ist jedoch auch bei einem HC-Sensor und einer λ-Sonde mit unterschiedlichen Querempfindlichkeiten bzw. Empfindlichkeitsbereichen entsprechend anwendbar.

## Patentansprüche

1. Verfahren zur Bestimmung des Gehalts einer vorgegebenen ersten Gaskomponente in einem Abgas einer Brennkraftmaschine (1) stromab eines in einer Abgasleitung (3) der Brennkraftmaschine (1) angeordneten Abgaskatalysators (4), wobei
- ein an ein elektronisches Steuergerät (7) angeschlossener erster Gassensor (5) von wenigstens einem Teil des aus dem Abgaskatalysator (4) austretenden Abgases angeströmt wird,
**dadurch gekennzeichnet, dass**
- ein an das elektronische Steuergerät (7) angeschlossener zweiter Gassensor (6) von wenigstens einem Teil des aus dem Abgaskatalysator (4) austretenden Abgases angeströmt wird und
- der Gehalt der ersten Gaskomponente vom Steuergerät (7) durch eine Verknüpfung eines Signals des ersten Gassensors (5) mit einem Signal des zweiten Gassensors (6) ermittelt wird.

2. Verfahren nach Anspruch 1,
**dadurch gekennzeichnet, dass** der erste Gassensor (5) und der zweite Gassensor (6) unterschiedliche Kennlinien aufweisen.

3. Verfahren nach Anspruch 1 oder 2,
**dadurch gekennzeichnet, dass** das Signal des ersten Gassensors (5) mit dem Partialdruck der ersten Gaskomponente korreliert und das Signal des zweiten Gassensors (6) mit dem Partialdruck einer zweiten, zur ersten Gaskomponente unterschiedlichen Gaskomponente korreliert.

4. Verfahren nach einem der vorangehenden Ansprüche,
**dadurch gekennzeichnet, dass** das Signal des ersten Gassensors (5) wenigstens mit dem Partialdruck von Stickoxid korreliert und das Signal des zweiten Gassensors (6) mit dem Partialdruck von Sauerstoff korreliert und vom Steuergerät (7) durch Verknüpfung des Signals des ersten Gassensors (5) mit dem Signal des zweiten Gassensors (6) der Stickoxidgehalt im Abgas ermittelt wird.

5. Verfahren nach einem der vorangehenden Ansprüche,
**dadurch gekennzeichnet, dass** die Verknüpfung der Signale der Gassensoren (5, 6) derart vorgenommen wird, dass unterhalb eines vorgebbaren Schwellenwertes S1 für das Signal des zweiten Gassensors (6) das Signal des ersten Gassensors (5) mit einem vorgebbaren ersten Faktor F1 multipliziert wird und oberhalb des Schwellenwertes S1 für das Signal des zweiten Gassensors (6) das Signal des ersten Gassensors (5) mit einem vorgebbaren zweiten Faktor F2 multipliziert wird.

6. Verfahren nach einem der vorangehenden Ansprüche,
**dadurch gekennzeichnet, dass** die Verknüpfung nur dann vorgenommen wird, wenn das Signal des ersten Gassensors (5) einen zumindest lokales Maximum überschritten hat.

7. Verfahren nach einem der vorangehenden Ansprüche,
**dadurch gekennzeichnet, dass** die Verknüpfung vorgenommen wird, wenn das Signal des zweiten Gassensors (6) einen Wert innerhalb eines vorgebbaren Wertebereichs aufweist.

8. Verfahren nach einem der vorangehenden Ansprüche,
**dadurch gekennzeichnet, dass** durch Integration des ermittelten Gehalts der ersten Gaskomponente über einen vorgebbaren Zeitbereich hinweg die Gesamtemission der ersten Gaskomponente in dem Zeitbereich ermittelt wird.

9. Verfahren nach einem Anspruch 8,
**dadurch gekennzeichnet, dass** bei Überschreiten eines vorgebbaren Schwellenwertes E1 für die Gesamtemission der ersten Gaskomponente vom Steuergerät (7) eine Meldung erzeugt wird.
